# EUROPEAN PATENT APPLICATION

(11) **EP 4 624 642 A1**
(43) Date of publication of application: **01.10.2025**
(21) Application number: 23891617.5
(22) Date of filing: 15.11.2023
(51) Int. Cl.: D01F 4/00, C07C 51/44, C07C 51/46, C07C 51/48, D01D 5/06, D01F 13/02, C07K 14/435

(54) **METHOD FOR PRODUCING SHAPED BODY CONTAINING PROTEIN, AND METHOD FOR RECOVERING CARBOXYLIC ACID**

(30) Priority: 16.11.2022 JP 2022183498
(71) Applicant: Spiber Inc., Tsuruoka-shi, Yamagata 997-0052 (JP); Refine Holdings Co., Ltd., Anpachi-gun Gifu 503-0212 (JP)
(72) Inventor: ISHII, Hideto, Tsuruoka-shi, Yamagata 997-0052 (JP); KOTAKA, Koichi, Tsuruoka-shi, Yamagata 997-0052 (JP); SATO, Ryoko, Tsuruoka-shi, Yamagata 997-0052 (JP); ANDO, Hirotada, Kariya-shi, Aichi 448-8651 (JP); FUJITA, Tomoya, Tokyo 100-0005 (JP); SUZUKI, Takao, Tokyo 100-0005 (JP); TAKEYAMA, Tomokiyo, Tokyo 100-0005 (JP); ISHIZAWA, Hideki, Tokyo 100-0005 (JP); ODA, Akiyoshi, Tokyo 100-0005 (JP); NAKASHIMA, Kouji, Tokyo 100-0005 (JP); NOUMI, Tadashi, Tokyo 100-0005 (JP)
(74) Representative: Dehns
(86) International application number: PCT/JP2023/041110
(87) International publication number: WO 2024/106470

(57) **Abstract**

An object of the present invention is to enable, when producing a formed article by a method including eluting a carboxylic acid in a protein solution containing a protein and the carboxylic acid into a coagulation liquid, efficient recovery of the carboxylic acid from the coagulation liquid. In order to achieve the object, the present invention provides a method for producing a formed article containing a protein, including: introducing a protein solution containing a carboxylic acid into a coagulation liquid; and recovering the carboxylic acid from the coagulation liquid, wherein recovering the carboxylic acid from the coagulation liquid includes: a first step of bringing the coagulation liquid into liquid-liquid contact with a prepared extractant containing an extraction solvent and a diluent to dissolve the carboxylic acid contained in the coagulation liquid into the prepared extractant, thereby forming a prepared extractant containing the carboxylic acid and water; a second step of forming a diluent layer and an aqueous layer while distilling off the diluent and water from the prepared extractant and also recovering the remaining prepared extractant containing the carboxylic acid; and a third step of obtaining a purified carboxylic acid through a distillation operation including distilling off the carboxylic acid from the prepared extractant.

## Description

### Technical Field

The present disclosure relates to a method for producing a formed article containing a protein and a method for recovering a carboxylic acid.

### Background Art

Formed articles obtained using biodegradable proteins are gaining significant attention as alternatives to those made of petroleum-derived materials. Various methods have been proposed for producing formed articles containing proteins.

For example, Patent Documents 1 and 2 each disclose a method for obtaining formed articles, such as fibers, including extruding a spinning solution containing a protein dissolved in a carboxylic acid into a coagulation liquid. According to such a method, the carboxylic acid eluted from the spinning solution dissolves into the coagulation liquid. From the perspective of environmental pollution control and resource reuse, it is desirable to recover the carboxylic acid from the coagulation liquid when discarding the coagulation liquid.

On the other hand, in technical fields unrelated to the production of formed articles containing a protein, various methods have been proposed for recovering a carboxylic acid from a carboxylic acid aqueous solution generated as waste liquid in various processes (e.g., Patent Documents 3 to 12).

### Prior Art Documents

### Patent Documents

[Patent Document 1] JP H4(1992)-263614A
[Patent Document 2] JP 2004-532362A
[Patent Document 3] JP H9(1997)-151158A
[Patent Document 4] JP 2018-062512A
[Patent Document 5] JP S61(1986)-176550A
[Patent Document 6] JP S61(1986)-176551A
[Patent Document 7] JP H08(1996)-283191A
[Patent Document 8] JP S61(1986)-176552A
[Patent Document 9] JP S61(1986)-176553A
[Patent Document 10] JP S55(1980)-154935A
[Patent Document 11] JP S61(1986)-043133A
[Patent Document 12] DE 2545658A1

### Disclosure of Invention

### Problem to be Solved by the Invention

Conventional methods for recovering a carboxylic acid are not necessarily suitable for use in the recovery of a carboxylic acid during the production of a formed article containing a protein in terms of productivity, production costs, and the like.

The present disclosure relates to a novel method that enables, when producing a formed article by a method including eluting a carboxylic acid in a protein solution containing a protein and the carboxylic acid into a coagulation liquid, efficient recovery of the carboxylic acid from the coagulation liquid. The present disclosure also relates to a novel method that enables, from a carboxylic acid aqueous solution containing a carboxylic acid that has been eluted from a protein solution containing a protein and a solvent containing the carboxylic acid, efficient recovery of the carboxylic acid.

### Means for Solving Problem

The present disclosure encompasses at least the following.
[1] A method for producing a formed article containing a protein, including:
   introducing a protein solution containing a protein and a solvent containing a carboxylic acid into a coagulation liquid X containing water;
   eluting the carboxylic acid in the protein solution into the coagulation liquid X, thereby forming a formed article containing the protein; and
   recovering the carboxylic acid from a coagulation liquid Y in which the formed article containing the protein has been formed.

Recovering the carboxylic acid from the coagulation liquid Y includes:
a first step of bringing the coagulation liquid Y into liquid-liquid contact with a prepared extractant I containing: an extraction solvent that dissolves the carboxylic acid and a diluent that is completely compatible with the extraction solvent, thereby forming a coagulation liquid Z and a prepared extractant II,
where in the coagulation liquid Z, a concentration of the extraction solvent and a concentration of the diluent are each less than 0.001 in terms of mass fraction and a concentration of the carboxylic acid is less than 0.005 in terms of mass fraction, and
in the prepared extractant II, 90 mass% or more of the carboxylic acid present in the coagulation liquid Y is dissolved and a concentration of water is less than 0.05 in terms of mass fraction;
a second step of forming a diluent layer containing the diluent and an aqueous layer containing water while azeotropically distilling off the diluent and the water from the prepared extractant II and also recovering a prepared extractant III that is a remainder containing the carboxylic acid; and
a third step of obtaining a purified carboxylic acid containing the carboxylic acid that has been purified through a distillation operation including distilling off the carboxylic acid from the prepared extractant III recovered in the second step.

The extraction solvent is a poorly water-soluble organic solvent having a boiling point higher than a boiling point of the diluent at atmospheric pressure.

The diluent is:
a first hydrophobic solvent that forms an azeotropic mixture with water, has a water concentration of 0.2 or more in terms of mass fraction and has a boiling point higher than a boiling point of water and a boiling point of the carboxylic acid at atmospheric pressure, wherein the first hydrophobic solvent does not form an azeotropic mixture with the carboxylic acid; or
a second hydrophobic solvent that forms an azeotropic mixture with water, has a water concentration of 0.2 or more in terms of mass fraction and has a boiling point higher than a boiling point of water and a boiling point of the carboxylic acid at atmospheric pressure,
   where the second hydrophobic solvent forms an azeotropic mixture with the carboxylic acid, and a mixed liquid containing the second hydrophobic solvent and the carboxylic acid at any ratio separates into a layer containing the second hydrophobic solvent and a layer containing the carboxylic acid.

When the diluent is the second hydrophobic solvent, in the third step, the distillation operation includes azeotropically distilling off the carboxylic acid and the diluent from the prepared extractant III, and the purified carboxylic acid and the diluent layer containing the diluent are formed by the distillation operation, and
in the purified carboxylic acid, a concentration of water, a concentration of the extraction solvent, and a concentration of the diluent are each less than 0.01 in terms of mass fraction.

[2] The method according to [1],
where in the first step, a distribution ratio D of the carboxylic acid represented by the following equation is 0.3 or more: D = (a concentration of the carboxylic acid in the prepared extractant II)/(the concentration of the carboxylic acid in the coagulation liquid Z).

[3] The method according to [1] or [2],
where a concentration of the carboxylic acid in the coagulation liquid Y is from 0.05 to 0.3 in terms of mass fraction, and
a concentration of the carboxylic acid in the purified carboxylic acid is 0.99 or more in terms of mass fraction.

[4] The method according to any one of [1] to [3],
where the diluent layer formed in the second step is added to the prepared extractant II.

[5] The method according to any one of [1] to [4],
where the aqueous layer formed in the second step is added to the coagulation liquid Y

[6] The method according to any one of [1] to [5],
where a prepared extractant IV remaining after the carboxylic acid has been distilled off from the prepared extractant III in the third step is added to the prepared extractant I.

[7] The method according to any one of [1] to [6],
where the diluent is the second hydrophobic solvent, and the diluent layer formed in the third step is added to the prepared extractant III.

[8] A method for recovering a carboxylic acid, including:
preparing a carboxylic acid aqueous solution Y containing a carboxylic acid, the carboxylic acid having been eluted from a protein solution containing a protein and a solvent containing the carboxylic acid and a concentration of the carboxylic acid being from 0.05 to 0.3 in terms of mass fraction; and
recovering the carboxylic acid from the carboxylic acid aqueous solution Y

Recovering the carboxylic acid from the carboxylic acid aqueous solution Y includes:
a first step of bringing the carboxylic acid aqueous solution Y into liquid-liquid contact with a prepared extractant I containing: an extraction solvent that dissolves the carboxylic acid and a diluent that is completely compatible with the extraction solvent, thereby forming a carboxylic acid aqueous solution Z and a prepared extractant II,
where in the carboxylic acid aqueous solution Z, a concentration of the extraction solvent and a concentration of the diluent are each less than 0.001 in terms of mass fraction, and a concentration of the carboxylic acid is less than 0.005 in terms of mass fraction, and
in the prepared extractant II, 90 mass% or more of the carboxylic acid present in the carboxylic acid aqueous solution Y is dissolved and a concentration of water is less than 0.05 in terms of mass fraction;
a second step of forming a diluent layer containing the diluent and an aqueous layer containing water while azeotropically distilling off the diluent and the water from the prepared extractant and also recovering a prepared extractant III that is a remainder containing the carboxylic acid; and
a third step of obtaining a purified carboxylic acid containing the carboxylic acid that has been purified through a distillation operation including distilling off the carboxylic acid from the prepared extractant III recovered in the second step.

The extraction solvent is a poorly water-soluble organic solvent having a boiling point higher than that of the diluent at atmospheric pressure.

The diluent is:
a first hydrophobic solvent that forms an azeotropic mixture with water, has a water concentration of 0.2 or more in terms of mass fraction and has a boiling point higher than a boiling point of water and a boiling point of the carboxylic acid at atmospheric pressure, where the first hydrophobic solvent does not form an azeotropic mixture with the carboxylic acid; or
a second hydrophobic solvent that forms an azeotropic mixture with water with a water concentration of 0.2 or more in terms of mass fraction and has a boiling point higher than a boiling point of water and a boiling point of the carboxylic acid at atmospheric pressure, where the second hydrophobic solvent forms an azeotropic mixture with the carboxylic acid, and a mixed liquid containing the second hydrophobic solvent and the carboxylic acid at any ratio separates into a layer containing the second hydrophobic solvent and a layer containing the carboxylic acid.

When the diluent is the second hydrophobic solvent, in the third step, the distillation operation includes azeotropically distilling off the carboxylic acid and the diluent from the prepared extractant III, and the purified carboxylic acid and the diluent layer containing the diluent are formed by the distillation operation, and
in the purified carboxylic acid, a concentration of water, a concentration of the extraction solvent, and a concentration of the diluent are each less than 0.01 in terms of mass fraction and a concentration of the carboxylic acid is 0.99 or more in terms of mass fraction.
[9] The method according to [8],
where in the first step, a distribution ratio D of the carboxylic acid represented by the following equation is 0.3 or more: D = (a concentration of the carboxylic acid in the prepared extractant II)/(the concentration of the carboxylic acid in the carboxylic acid aqueous solution Z).
[10] The method according to [8] or [9],
where the diluent layer formed in the second step is added to the prepared extractant II.
[11] The method according to any one of [8] to [10],
where the aqueous layer formed in the second step is added to the carboxylic acid aqueous solution Y
[12] The method according to any one of [8] to [11],
where a prepared extractant IV remaining after the carboxylic acid has been distilled off from the prepared extractant III in the third step is added to the prepared extractant I.
[13] The method according to any one of [8] to [12],
where the diluent is the second hydrophobic solvent, and the diluent layer formed in the third step is added to the prepared extractant III.

### Effects of the Invention

The present disclosure can provide a novel method that enables, when producing a formed article by a method including eluting a carboxylic acid in a protein solution containing a protein and the carboxylic acid into a coagulation liquid, efficient recovery of the carboxylic acid from the coagulation liquid. The present disclosure can also provide a novel method that enables, from a carboxylic acid aqueous solution containing a carboxylic acid that has been eluted from a protein solution containing a protein and a solvent containing the carboxylic acid, efficient recovery of the carboxylic acid.

### Brief Description of Drawings

[FIG. 1] FIG. 1 is a schematic diagram illustrating an example of a spinning apparatus for producing a formed article.
[FIG. 2] FIG. 2 is a schematic diagram illustrating an example of a method for recovering a carboxylic acid from a coagulation liquid.

### Description of the Invention

The following examples should not be construed as limiting the present invention.

### Method for Producing Formed article

One example of a method for producing a formed article containing a protein includes: introducing a protein solution containing a protein and a solvent containing a carboxylic acid into a coagulation liquid X containing water, eluting the carboxylic acid in the protein solution into the coagulation liquid X, thereby forming a formed article containing the protein; and recovering the carboxylic acid from a coagulation liquid Y in which the formed article containing the protein has been formed.

There is no particular limitation on the formed article, and the formed article may be in any form and size. For example, the formed article may be in the form of a fiber, tube, rod, or granule. Alternatively, the formed article may be a gel. An elongated formed article, e.g., a fiber, can be produced by, for example, extruding a protein solution into a coagulation liquid through a nozzle hole. A granule can be produced by, for example, dripping a protein solution into a coagulation liquid. In addition to the above examples, any method for producing a formed article using a coagulation liquid can be employed to produce any desired formed article.

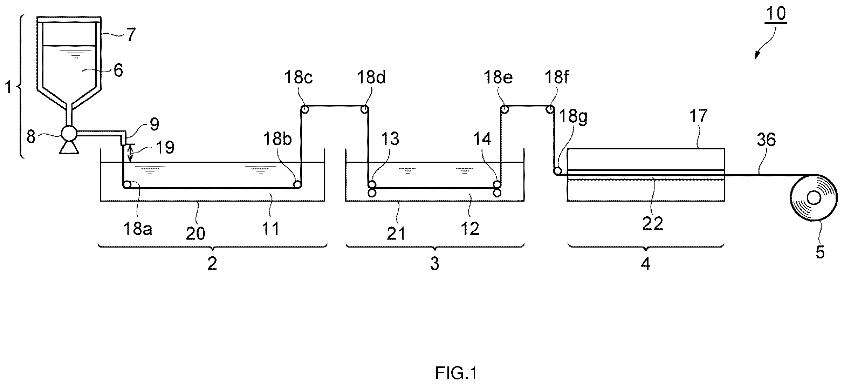

FIG. 1 is a schematic diagram illustrating an example of a spinning apparatus for producing a fiber, which is an example of the formed article. A spinning apparatus 10 shown in FIG. 1 is an example of a spinning apparatus for dry and wet spinning, and includes an extruder 1, a coagulation device 2, a washing device 3, and a drying device 4, in this order, from the upstream side.

The extruder 1 has a storage tank 7, and a protein solution 6 used as a spinning dope is stored in this storage tank 7. A coagulation liquid 11 is stored in a coagulation bath 20. By a gear pump 8 attached to a lower end part of the storage tank 7, the protein solution 6 is extruded through a nozzle 9 that is provided spaced apart from the coagulation liquid 11 with an air gap 19 between them. The extruded protein solution 6 is introduced into the coagulation bath 20 of the coagulation device 2 after passing through the air gap 19. In the coagulation liquid 11 stored in the coagulation bath 20, a solvent containing a carboxylic acid is eluted from the protein solution into the coagulation liquid 11, whereby a protein is coagulated. A fiber (formed article) containing the coagulated protein is then guided to a washing bath 21 of the washing device 3, washed with a washing liquid 12 in the washing bath 21, and then fed to the drying device 4 by a first nip roller 13 and second nip roller 14 provided in the washing bath 21. At this time, for example, by setting the rotational speed of the second nip roller 14 higher than that of the first nip roller 13, a fiber 36 (formed article) stretched at a stretch ratio corresponding to the rotational speed ratio is obtained. After exiting the washing bath 21, the fiber, stretched in the washing liquid 12, is dried as it passes through a yarn path 22 provided in a drying oven 17 of the drying device 4, and then wound onto a winder. In the above-described manner, with the spinning apparatus 10, the fiber (formed article) 36 is obtained finally in the form of a wound roll 5 wound onto the winder. The spinning apparatus 10 also includes yarn guides 18a to 18g. The spinning apparatus 10 may include a plurality of coagulation baths for storing a coagulation liquid.

The content of the protein in the protein solution 6 may be 1 mass% or more, 2 mass% or more, 4 mass% or more, 7 mass% or more, 10 mass% or more, 15 mass% or more, 20 mass% or more, 25 mass% or more, or 30 mass% or more and may be 50% or less, 45 mass% or less, 40 mass% or less, or 35 mass% or less with respect to the total amount of the protein solution. The content of the protein in the protein solution 6 may be from 1 to 50 mass%, from 2 to 45 mass%, from 4 to 45 mass%, from 7 to 45 mass%, from 10 to 40 mass%, from 15 to 40 mass%, from 20 to 40 mass%, from 25 to 35 mass%, or from 30 to 35 mass% with respect to the total amount of the protein solution. Examples of the protein that may be included in the protein solution 6 will be described below.

The protein solution 6 mainly contains a carboxylic acid as a solvent. The carboxylic acid may be, for example, a carboxylic acid having 1 to 3 carbon atoms. The carboxylic acid may be formic acid (boiling point: 100.8°C), acetic acid (boiling point: 118.0°C), propionic acid (boiling point: 141.2°C), or a combination thereof. In one non-limiting example, the carboxylic acid may be, for example, formic acid. The boiling points of the above-described substances and the boiling points of substances to be described below are values at ordinary pressure (101.325 kPa) and vary depending on the pressure condition at the time of measurement.

The content of the solvent in the protein solution 6 may be 50 parts by mass or more, 60 parts by mass or more, 65 parts by mass or more, 70 parts by mass or more, or 80 parts by mass or more and may be 90 parts by mass or less, 85 parts by mass or less, 80 parts by mass or less, 75 parts by mass or less, 70 parts by mass or less, 65 parts by mass or less, 60 parts by mass or less, or 50 parts by mass or less with respect to 100 parts by mass of the protein solution. The content of the solvent in the protein solution 6 may be from 60 to 90 parts by mass, from 60 to 85 parts by mass, from 65 to 85 parts by mass, from 70 to 80 parts by mass, or from 70 to 75 parts by mass with respect to 100 parts by mass of the protein solution. The content of the carboxylic acid in the protein solution 6 may be from 60 to 90 parts by mass, from 60 to 85 parts by mass, from 65 to 85 parts by mass, from 70 to 80 parts by mass, or from 70 to 75 parts by mass with respect to 100 parts by mass of the protein solution.

The coagulation liquid 11 (coagulation liquid X) is a liquid containing water and capable of dissolving the carboxylic acid contained in the protein solution 6. The coagulation liquid 11 may contain a lower alcohol having 1 to 5 carbon atoms or other solvents, e.g., acetone. Examples of the lower alcohol include methanol, ethanol, and 2-propanol.

The coagulation liquid X may contain an inorganic salt. The inorganic salt may be, for example, at least one selected from the group consisting of carboxylates, sulfates, chlorides, nitrates, iodide salts, carbonates, hydrogen sulfates, hydrogen phosphates, hydrogencarbonates, and thiocyanates.

When the coagulation liquid X contains an inorganic salt (e.g., sodium sulfate), separation into layers is likely to occur properly in the liquid-liquid contact in the first step, which will be described below. If separation into layers occurs properly, water is unlikely to enter the prepared extractant side. From this viewpoint, the concentration (mass fraction) of an inorganic salt in the coagulation liquid 11 may be 0.003 or more and 0.2 or less, or 0.1 or more and 0.15 or less.

The temperature of the coagulation liquid X is not limited to a particular temperature, and may be 40°C or less, 30°C or less, 25°C or less, 20°C or less, 10°C or less, or 5°C or less, and may be 0°C or more.

After leaving the coagulation bath or the washing bath, the fiber (formed article) containing the coagulated protein may be directly wound onto the winder, or may pass through the drying device to be dried and then be wound onto the winder.

The method for producing a formed article may further include stretching the formed article. In the example illustrated in FIG. 1, the fiber as the formed article may be stretched, for example, in the coagulation bath 20 or the washing bath 21. Alternatively, the formed article may be stretched in the air.

The coagulation liquid 11 after the fiber 36 as the formed article has been formed (coagulation liquid Y) is a carboxylic acid aqueous solution containing the carboxylic acid that has been eluted from the protein solution and water. The concentration of the carboxylic acid in the coagulation liquid Y may be, for example, from 0.05 to 0.3 in terms of mass fraction. As used herein, the term "mass fraction" refers to the concentration expressed by the mass ratio relative to the total mass.

The method for recovering the carboxylic acid from the coagulation liquid Y includes, for example, a first step of bringing the coagulation liquid Y into liquid-liquid contact with a prepared extractant I containing an extraction solvent and a diluent to cause the carboxylic acid contained in the coagulation liquid Y to dissolve into the prepared extractant I, thereby forming a prepared extractant II containing the carboxylic acid and water; a second step of forming a diluent layer containing the diluent and an aqueous layer containing water while azeotropically distilling off the diluent and the water from the prepared extractant II and also recovering a prepared extractant III that is a remainder containing the carboxylic acid; and a third step of obtaining a purified carboxylic acid through a distillation operation including distilling off the carboxylic acid from the prepared extractant III.

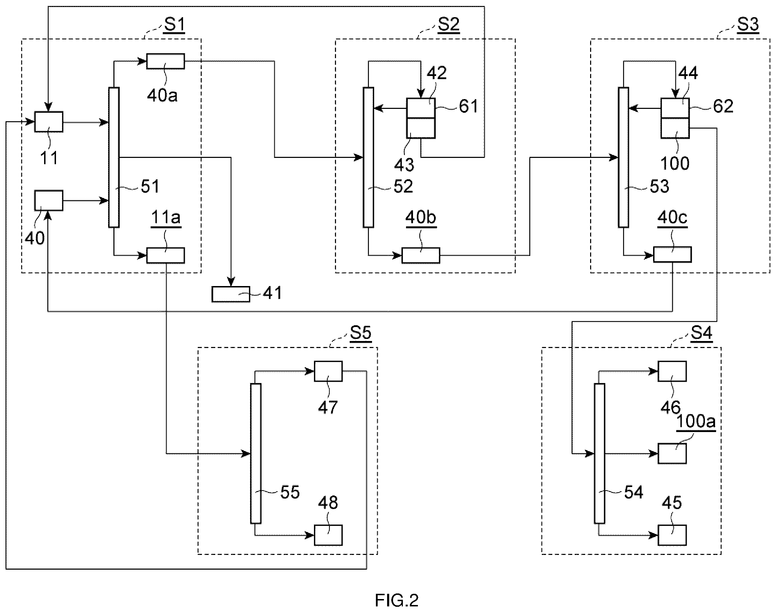

FIG. 2 is a schematic diagram illustrating an example of the method for recovering the carboxylic acid from the coagulation liquid Y In a first step S1 of the method illustrated in FIG. 2, a coagulation liquid 11 (coagulation liquid Y) - a carboxylic acid aqueous solution containing a carboxylic acid and water - and a prepared extractant 40 (prepared extractant I) containing an extraction solvent and a diluent, are mixed together in a liquid-liquid extraction section 51. Through liquid-liquid contact between the coagulation liquid 11 and the prepared extractant 40 in the liquid-liquid extraction section 51, the carboxylic acid in the coagulation liquid 11 is extracted into the prepared extractant 40. In the liquid-liquid contact in the first step, a continuous phase may be either the coagulation liquid 11 or the prepared extractant 40. When the coagulation liquid 11 contains impurities, the impurities may be removed from the coagulation liquid 11 beforehand by filtration or the like.

The liquid-liquid extraction section 51 may be, for example, a packed tower commonly used for liquid-liquid extraction. Typically, a prepared extractant 40a (prepared extractant II), which is a light liquid containing the carboxylic acid and water, is extracted from an upper part of the liquid-liquid extraction section 51, while a coagulation liquid 11a (coagulation liquid Z), which is a heavy liquid, is extracted from a lower part of the liquid-liquid extraction section 51. The coagulation liquid 11a may be added to the coagulation liquid 11 (coagulation liquid Y) before being brought into liquid-liquid contact with the prepared extractant 40. If impurities precipitate at the interface in the liquid-liquid contact, a middle layer 41 containing the impurities may be removed from the liquid-liquid extraction section 51. The impurities may be extracted from the lower part of the liquid-liquid extraction section 51 together with the coagulation liquid 11a. The impurities may be removed by centrifuging the extracted mixture containing the impurities. The liquid-liquid extraction section for the first step is not limited to a packed tower utilizing a static liquid-liquid contact mechanism, and may be, for example, an extraction device utilizing a dynamic liquid-liquid contact mechanism, e.g., an RDC (rotating disk contactor-type countercurrent continuous extractor) or a Karr column.

In the coagulation liquid 11a (coagulation liquid Z) extracted from the liquid-liquid extraction section 51, the concentrations of the extraction solvent and the diluent may each be less than 0.001 in terms of mass fraction. In the coagulation liquid 11a, the concentration of the carboxylic acid may be less than 0.005 in terms of mass fraction. When the coagulation liquid 11 (coagulation liquid Y) before being introduced into the liquid-liquid extraction section 51 contains an inorganic salt, the concentration of the inorganic salt in the coagulation liquid 11a extracted from the liquid-liquid extraction section 51 may be 0.003 or more and less than 0.2 in terms of mass fraction. The coagulation liquid 11a in which the concentrations of the respective components are within these ranges can be formed easily by, for example, appropriately selecting the composition of the prepared extractant 40.

Through the liquid-liquid contact between the coagulation liquid and the prepared extractant, the majority of the carboxylic acid in the coagulation liquid 11 (coagulation liquid Y) can be dissolved in the prepared extractant 40 while suppressing the migration of the water into the prepared extractant 40 (prepared extractant I). For example, the proportion of the amount of the carboxylic acid contained in the prepared extractant 40a (prepared extractant II) after the first step with respect to the amount of the carboxylic acid contained in the coagulation liquid 11 before the first step (i.e., recovery rate) may be 90 mass% or more. The concentration of water in the prepared extractant 40a after the first step may be less than 0.05 in terms of mass fraction. When the coagulation liquid 11 contains an inorganic salt, the concentration of the inorganic salt in the prepared extractant 40a after the first step may be less than 0.0001 in terms of mass fraction. The prepared extractant 40a in which the concentrations of the respective components are within these ranges can be obtained easily by, for example, appropriately selecting the composition of the prepared extractant 40.

The temperature of the coagulation liquid 11 and the prepared extractant 40 (the temperature thereof in the liquid-liquid extraction section 51) subjected to the liquid-liquid contact may be, for example, from 10°C to 90°C, or from 30°C to 50°C. When the coagulation liquid 11 contains an inorganic salt, precipitation of the inorganic salt at the interface of the liquid-liquid contact can be suppressed easily by setting the temperature to fall within the above-described range.

The diluent contained in the prepared extractant may be a hydrophobic solvent. The solubility of the hydrophobic solvent in water at 25°C may be less than 0.01 or less than 0.001 in terms of mass fraction. In the extraction through liquid-liquid contact in the first step, the diluent can contribute to improving the layer separability and suppressing the migration of water to the prepared extractant.

The diluent may be a hydrophobic solvent that forms an azeotropic mixture with water and has a boiling point higher than those of water and the carboxylic acid at atmospheric pressure. The azeotropic mixture of the diluent and water may be a minimum boiling azeotropic mixture. In the azeotropic mixture of the diluent and water, the concentration (mass fraction) of the water may be 0.2 or more, or 0.5 or more. As used herein, the term "at atmospheric pressure" means being in an atmosphere of, for example, 101.3 ± 2 kPa. A hydrophobic solvent that forms an azeotropic mixture with water serves to push up water to the top of a distillation tower preferentially over the carboxylic acid, i.e., serves as an azeotropic agent with water, in the second step, and as a result, it can improve the separation efficiency of the carboxylic acid.

The hydrophobic solvent as the diluent may be a first hydrophobic solvent that does not form an azeotropic mixture with the carboxylic acid. Alternatively, the hydrophobic solvent as the diluent may be a second hydrophobic solvent that forms an azeotropic mixture with the carboxylic acid and causes separation of a layer containing the hydrophobic solvent and a layer containing the carboxylic acid. The azeotropic mixture of the diluent and the carboxylic acid may be a minimum boiling azeotropic mixture. In the layer containing the carboxylic acid resulting from azeotropic distillation of the azeotropic mixture of the diluent and the carboxylic acid, the concentration (mass fraction) of the diluent (second hydrophobic solvent) may be less than 0.002.

The hydrophobic solvent as the diluent may be a hydrocarbon having a boiling point of 110°C to 220°C at atmospheric pressure. Examples of the hydrophobic solvent as a component of the diluent include saturated or unsaturated aliphatic hydrocarbons, and aromatic hydrocarbons. The diluent may be at least one hydrophobic solvent selected from the group consisting of toluene, octane, isooctane, nonane, decane, undecane, dodecane, o-xylene, m-xylene, p-xylene, and ethylbenzene. For example, the diluent may be toluene, octane, decane, or a combination thereof.

The extraction solvent contained in the prepared extractant 40 is a poorly water-soluble organic solvent that dissolves the carboxylic acid. The solubility of the poorly water-soluble extraction solvent in water at 25°C may be less than 0.01, e.g., less than 0.001, in terms of mass fraction. The extraction solvent may be an organic solvent that forms a single uniform phase without causing separation from the diluent at the temperature of the coagulation liquid and the prepared extractant in the liquid-liquid extraction section 51 used in the first step S1. In other words, the extraction solvent may be an organic solvent that is completely compatible with the diluent.

The extraction solvent may have a boiling point higher than that of the diluent at atmospheric pressure. The extraction solvent having a high boiling point and high affinity for the carboxylic acid can suppress vaporization of the carboxylic acid during a distillation operation in the second step. As a result, water tends to be distilled off preferentially. The boiling point of the extraction solvent may be from 100°C to 300°C or from 140°C to 300°C at atmospheric pressure. The boiling point of the extraction solvent may be from 140°C to 280°C in the pressure range of from 6.67 kPa to 66.7 kPa.

The extraction solvent may be selected based on the distribution ratio of the carboxylic acid. The distribution ratio D of the carboxylic acid is expressed by, for example, the following equation: D = (a concentration of the carboxylic acid in the extraction solvent)/(a concentration of the carboxylic acid in water). An extraction solvent exhibiting a high distribution ratio D allows more efficient recovery of the carboxylic acid. For example, an extraction solvent in which the distribution ratio D of the carboxylic acid is 0.3 or more or 0.6 or more at the same temperature as the temperature of the coagulation liquid and the prepared extractant in the liquid-liquid extraction section 51 in the first step S1 may be selected.

The extraction solvent may be, for example, an organophosphorus compound, an amide compound, or a combination thereof. Examples of the organophosphorus compound include tributyl phosphate and trioctylphosphine oxide. Examples of the amide compound include N,N-din-butylformamide and N-n-butyl-N-2 -ethylhexylformamide. The extraction solvent may be, for example, tributyl phosphate, N,N-di-n-butylformamide, or a combination thereof.

The combination of the extraction solvent and the diluent is selected considering the compatibility, the boiling point, and the like. For example, the extraction solvent may be tributyl phosphate, and the diluent may be toluene, octane, or decane. Alternatively, the extraction solvent may be N,N-di-n-butylformamide, and the diluent may be octane or decane.

The mass ratio between the extraction solvent and the diluent (extraction solvent : diluent) in the prepared extractant I may be from 1:5 to 9:1, from 1:1 to 5:1, or from 2:1 to 3:1. The mass ratio between the extraction solvent and the diluent (extraction solvent : diluent) in the prepared extractant may be 2:1, 2.1:1, 2.2:1, 2.3:1, 2.4:1, 2.5:1, 2.55:1, 2.6:1, 2.7:1, 2.8:1, 2.9:1, or 3:1. A moderately high ratio of the extraction solvent tends to improve the extraction efficiency of the carboxylic acid in the first step. A moderately high ratio of the diluent tends to allow recovery of the carboxylic acid with a particularly high yield through azeotropic distillation of water and the diluent in the second step. Also, a moderately high ratio of the diluent having a relatively low boiling point tends to moderately lower the boiling point at the tower bottom in the distillation operation of the third step. When the temperature at the tower bottom is low, corrosion in the tower caused by the carboxylic acid and gas generation due to decomposition of the carboxylic acid tend to be suppressed more easily.

In the first step S1, the distribution ratio D of the carboxylic acid as expressed by the following equation is preferably 0.3 or more or 0.6 or more from the viewpoint of improving the extraction efficiency of the carboxylic acid: D = (a concentration of the carboxylic acid in the prepared extractant II)/( a concentration of the carboxylic acid in the coagulation liquid Z).

In the second step S2, the prepared extractant 40a (prepared extractant II) obtained after the first step, which contains the extraction solvent, the diluent, the carboxylic acid, and water, is introduced into a distillation tower 52. In the distillation tower 52, a mixture containing the diluent and the water is fed from an upper part of the distillation tower 52 to a decanter 61 while azeotropically distilling off the diluent and the water from the prepared extractant 40a. In the decanter 61, a diluent layer 42 mainly containing the diluent and an aqueous layer 43 mainly containing the water are formed. The diluent layer 42 may contain a slight amount of the carboxylic acid and/or a slight amount of the extraction solvent. The diluent layer 42 may be returned to the distillation tower 52 and mixed with the prepared extractant 40a having been introduced to the distillation tower 52. From a lower part of the distillation tower 52, a prepared extractant 40b (prepared extractant III) remaining after the diluent and the water have been distilled off and containing the carboxylic acid, is recovered. The aqueous layer 43 may contain a slight amount of the carboxylic acid. The aqueous layer 43 may be added to the coagulation liquid 11 (coagulation liquid Y) before being brought into liquid-liquid contact with the prepared extractant 40 in the first step S1.

In the distillation operation for distilling off the diluent and the water in the second step, the degree of pressure reduction may be, for example, from 6.67 to 66.7 kPa, or from 13.3 to 26.7 kPa. Distillation under reduced pressure lowers the tower bottom temperature, thereby allowing decomposition of the carboxylic acid and corrosion in the tower to be suppressed easily. The distillation tower 52 may be, for example, a plate tower provided with from 5 to 10 plates. The reflux ratio may be about 1 to 3.

If the composition balance in the distillation tower 52 is disturbed, a prepared extractant - different from the prepared extractant 40a (prepared extractant II) -may be fed to the distillation tower 52 from any of plates positioned above a feed plate to which the prepared extractant 40a is introduced. Owing to the extracting effect of the extraction solvent, the carboxylic acid moves to the lower part of the distillation tower 52, whereby the loss of the carboxylic acid can be further reduced. The prepared extractant to be fed separately from the prepared extractant 40a may be either the prepared extractant I or a prepared extractant 40c (prepared extractant IV) recovered in the third step.

The decanter 61 for the second step may be, for example, a device that separates a mixed liquid into two layers utilizing an inclination.

When the coagulation liquid 11 (coagulation liquid Y) contains an inorganic salt, the inorganic salt may precipitate on the bottom of the distillation tower 52. In order to remove the precipitated inorganic salt, a filter section may be provided on a line for feeding the prepared extractant 40b (prepared extractant III) to the third step.

In the third step S3, the prepared extractant 40b (prepared extractant III) containing the extraction solvent, the diluent, and the carboxylic acid is introduced into a distillation tower 53. In the distillation tower 53, a distillation operation including distilling off the carboxylic acid from the prepared extractant 40b is performed, thereby forming a purified carboxylic acid 100 containing the carboxylic acid that has been purified. The third step S3 in FIG. 2 is an example where the diluent is, in particular, the second hydrophobic solvent. The carboxylic acid and the diluent are azeotropically distilled off from the prepared extractant 40b. A mixture containing the distilled carboxylic acid and diluent is introduced into a decanter 62. In the decanter 62, a diluent layer 44 mainly containing the diluent and a layer of the purified carboxylic acid 100 containing the carboxylic acid that has been purified are formed. From a lower part of the distillation tower 53, a prepared extractant 40c (prepared extractant IV) remaining after the carboxylic acid has been distilled off is extracted. The extracted prepared extractant 40c may be added to the prepared extractant 40 (prepared extractant I) before being brought into liquid-liquid contact with the coagulation liquid 11 (coagulation liquid Y) in the first step.

In the distillation operation for distilling off the carboxylic acid in the third step, the degree of pressure reduction may be, for example, from 6.67 to 66.7 kPa, or from 13.3 to 26.7 kPa. Distillation under reduced pressure lowers the temperature required for distillation, thereby allowing decomposition of the carboxylic acid and corrosion in the tower to be suppressed easily. This also allows the prepared extractant 40c with less impurities to be obtained easily, and this is advantageous for reusing the prepared extractant 40c (prepared extractant IV) in the first step. The distillation tower 53 may be, for example, a plate tower provided with 5 to 10 plates. The reflux ratio may be about 3 to 5.

The diluent layer 44 may contain a slight amount of the carboxylic acid and/or a slight amount of the extraction solvent. The diluent layer 44 may be returned to the distillation tower 53 and mixed with the prepared extractant 40b (prepared extractant III) in the distillation tower 53. The diluent layer 44 may be fed to the distillation tower 53 from any of the plates positioned below a feed plate to which the prepared extractant 40b is introduced. By introducing the diluent layer 44, it is possible to facilitate azeotropic distillation of the carboxylic acid and the diluent.

The decanter 62 for the third step may be, for example, a device that separates a mixed liquid into two layers utilizing an inclination.

A purified carboxylic acid can be obtained efficiently by combining the removal of the water from the prepared extractant through distillation in the second step and the removal of the carboxylic acid from the prepared extractant through re-distillation in the third step. By the presence of the diluent at the bottom of the distillation tower during distillation, an increase in the tower bottom temperature is suppressed. Accordingly, the heat resistance requirements for the material constituting the distillation tower may be less strict, and this allows the material to be selected from a broader range.

In the purified carboxylic acid 100 obtained in the third step S3, the concentrations of the water, the extraction solvent, and the diluent may each be less than 0.01 in terms of mass fraction. In the purified carboxylic acid 100, the concentration of the carboxylic acid may be 0.99 or more in terms of mass fraction.

The method for recovering the carboxylic acid from the coagulation liquid 11 (coagulation liquid Y) may further include a fourth step S4 of removing, from the purified carboxylic acid 100 obtained in the third step, remaining water and the like by a distillation operation. By performing an additional distillation operation, the degree of purification of the carboxylic acid can be further improved. In the fourth step, the distillation operation may be performed once, or two or more times. In a purified carboxylic acid 100a obtained after the fourth step S4, the concentration (mass fraction) of the carboxylic acid may be 0.99 or more, 0.995 or more, or 0.0998 or more, and the concentration (mass fraction) of the water may be 0.002 or less.

For the fourth step S4, the purified carboxylic acid 100 is introduced into a distillation tower 54. A heavy liquid 45 containing the water is extracted from a lower part of the distillation tower 54, and the purified carboxylic acid 100a is extracted from a central part of the distillation tower 54. The heavy liquid 45 may contain the extraction solvent. A light liquid 46 containing the diluent may be extracted from an upper part of the distillation tower 54. The light liquid 46 may contain the carboxylic acid. Since the water and the extraction solvent are concentrated at the bottom of the distillation tower 54 and the diluent is concentrated at the top of the distillation tower 54, it is possible to recover the purified carboxylic acid 100a with a high degree of purity.

In the distillation operation in the fourth step, the degree of pressure reduction may be, for example, from 6.67 to 66.7 kPa, or from 26.7 to 40.0 kPa. The distillation tower 54 may be, for example, a plate tower provided with 5 to 10 plates. The reflux ratio may be about 3 to 5.

The method for recovering the carboxylic acid from the coagulation liquid 11 (coagulation liquid Y) may further include a fifth step S5 of distilling off the diluent, the extraction solvent, and the carboxylic acid from the coagulation liquid 11a (coagulation liquid Z) recovered in the first step S1 to form a distillate 47 containing them, and also recovering a mixed liquid 48 remaining after the distillation.

For the fifth step S5, the coagulation liquid 11a (coagulation liquid Z) is introduced into a distillation tower 55. Through distillation or stripping in the distillation tower 55, the diluent, the extraction solvent, and the carboxylic acid are distilled off, and the distillate 47 containing them is recovered from an upper part of the distillation tower 55. The distillate 47 may be added to the coagulation liquid 11 (coagulation liquid Y) before being brought into liquid-liquid contact with the prepared extractant 40 (prepared extractant I). The mixed liquid 48 is recovered from a lower part of the distillation tower 55. When the coagulation liquid 11a (coagulation liquid Z) contains an inorganic salt, the mixed liquid 48 normally contains the inorganic salt as well. The fifth step enables not only recovery of the carboxylic acid but also reuse of the inorganic salt.

The distillation or stripping treatment in the fifth step can be, for example, performed at atmospheric pressure (about 101.3 ± 2 kPa). The distillation tower 55 may be, for example, a plate tower provided with 5 to 10 plates.

### Protein

Examples of a protein that may be contained in a protein solution used for producing a formed article will be described below.

The protein may be either a natural protein or an artificial protein. The protein may be, for example, a protein that is industrially applicable. Being "industrially applicable" means that, for example, the protein can be used as a wide variety of general-purpose materials and the like for indoor and outdoor use. The industrially applicable protein may be, for example, a structural protein.

As used herein, the term "artificial protein" refers to an artificially produced protein, and examples thereof include recombinant proteins and synthetic proteins. The domain sequence of the artificial protein may be different from or identical to the amino acid sequence of a naturally occurring protein. The artificial protein may be a protein that utilizes the amino acid sequence of a naturally occurring protein as is, or a protein having an amino acid sequence modified based on the amino acid sequence of a naturally occurring protein (e.g., a protein having an amino acid sequence modified by altering the gene sequence of a cloned naturally occurring protein), or a protein that is artificially designed and synthesized without relying on a naturally occurring protein (e.g., a protein having an amino acid sequence formed by chemically synthesizing a nucleic acid encoding a designed amino acid sequence). By appropriately designing the amino acid sequence of the artificial protein, the function, properties, physical properties, and the like of the formed article can be controlled as desired. Since uniform molecular design is always possible for artificial proteins, it becomes possible to stably obtain a protein that is highly homologous to a target protein and suitable for an intended use. A formed article containing an artificial protein can be advantageous in terms of quality stabilization.

The number of amino acid residues in the protein may be, for example, 50 or more, 100 or more, 150 or more, 200 or more, 250 or more, 300 or more, 350 or more, 400 or more, 450 or more, or 500 or more. The number of amino acid residues in the protein may be, for example, 5000 or less, 4500 or less, 4000 or less, 3500 or less, 3000 or less, 2500 or less, 2000 or less, 1500 or less, or 1000 or less. The number of amino acid residues in the protein may be, for example, from 50 to 5000, from 100 to 4500, from 150 to 4000, from 200 to 3500, from 250 to 3000, from 300 to 2500, from 350 to 2000, from 400 to 1500, from 450 to 1000, or from 500 to 1000. The smaller the number of amino acid residues, the higher the solubility of the protein in a solvent tends to be.

The molecular weight of the protein may be, for example, from 2 to 500 kDa. The molecular weight of the protein may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more, and may be 500 kDa or less, 400 kDa or less, less than 360 kDa, 300 kDa or less, or 200 kDa or less.

The protein may be a structural protein. Structural proteins are a type of industrially applicable protein, and examples thereof include proteins involved in the structure of a living organism, proteins constituting a structure created by a living organism, and proteins derived therefrom. Structural proteins can self-aggregate under certain conditions, thereby forming a structure, e.g., a fiber, film, resin, gel, micelle, or nanoparticle. In general, structural proteins have a characteristic amino acid sequence or include a structure in which a motif composed of a few amino acid residues is repeated. Examples of natural structural proteins include fibroin (e.g., spider silk and silkworm silk), keratin, collagen, elastin, and resilin.

The structural protein may be an artificial structural protein. As used herein, the term "artificial structural protein" refers to an artificially produced structural protein. The artificial structural protein may be a structural protein produced from a microorganism utilizing genetic recombination technology, a protein having the same amino acid sequence as a natural structural protein, or a protein having a modified amino acid sequence improved from the viewpoint of productivity, formability, or the like.

Amino acids having a relatively small side chain are prone to form hydrogen bonds, and proteins containing a large amount of such amino acid residues thus form a formed article with higher strength more easily. Alanine and glycine residues, which have a non-polar side chain, are prone to be arranged to face inward during the folding process in polypeptide formation, whereby the resulting proteins are prone to have an α-helix structure or β-sheet structure. Accordingly, from the viewpoint of improving the strength of the formed article, it is desirable that the protein includes high proportions of glycine residues and alanine residues. The content of alanine residues in the protein may be, for example, from 10% to 40%, from 12% to 40%, from 15% to 40%, from 18% to 40%, from 20% to 40%, or from 22% to 40%. The content of glycine residues in the protein need only be 10% to 55%, for example, and may be from 11% to 55%, from 13% to 55%, from 15% to 55%, from 18% to 55%, from 20% to 55%, from 22% to 55%, or from 25% to 55%.

As used herein, the term "content of alanine residues" means the proportion of the number of alanine residues with respect to the number of all the amino acid residues constituting a protein, and is a value expressed by the following equation. Content of alanine residues = (the number of alanine residues in a protein/the number of all amino acid residues in the protein) × 100 (%)

The contents of glycine residues, serine residues, threonine residues, proline residues, and tyrosine residues are defined by the above equation in which the term "alanine residues" is replaced with the terms "glycine residues", "serine residues", "threonine residues", "proline residues", and "tyrosine residues", respectively.

The structural protein may include a certain degree of amino acid residues having a relatively large side chain or flexible amino acid residues distributed uniformly throughout its sequence. For example, the structural protein may include a plurality of motifs containing amino acid residues selected from tyrosine residues, threonine residues, and proline residues, and such motifs may be repeated periodically. A formed article containing such a structural protein tends to have favorable processability because strong intermolecular hydrogen bonds are unlikely to be formed during processing. For example, the proportion of the total number of proline residues, threonine residues, and tyrosine residues in any 20 contiguous amino acid residues may be 5% or more, more than 5.5%, 6.0% or more, more than 6.5%, 7.0% or more, more than 7.5%, 8.0% or more, more than 8.5%, 9.0% or more, 10.0% or more, or 15.0% or more. The proportion of the total number of proline residues, threonine residues, and tyrosine residues in any 20 contiguous amino acid residues may be 50% or less, 40% or less, 30% or less, or 20% or less. The proportion of the total number of proline residues, threonine residues, and tyrosine residues in any 20 contiguous amino acid residues may be from 5% to 50%, from 6.0% to 50%, from 7.0% to 40%, from 8.0% to 40%, 9.0% to 30%, from 10.0% to 30%, or from 15.0% to 25%.

The artificial structural protein may have repetitive sequences. In other words, the artificial structural protein may have a plurality of amino acid sequences (repetitive sequence units) that share high sequence identity with each other. The number of amino acid residues in each repetitive sequence unit may be 6 to 200. The sequence identity between the repetitive sequence units may be, for example, 85% or more, 90% or more, 95% or more, 96% or more, 97% or more, 98% or more, or 99% or more. The hydrophobicity (hydropathy index) of the repetitive sequence units may be, for example, -0.80 or more, -0.70 or more, -0.60 or more, -0.50 or more, -0.40 or more, -0.30 or more, -0.20 or more, -0.10 or more, 0.00 or more, 0.22 or more, 0.25 or more, 0.30 or more, 0.35 or more, 0.40 or more, 0.45 or more, 0.50 or more, 0.55 or more, 0.60 or more, 0.65 or more, or 0.70 or more. The hydrophobicity of the repetitive sequence units may be, for example, 1.0 or less, or 0.7 or less. The hydrophobicity of the repetitive sequence units may be, for example, -0.80 to 1.0.

The artificial structural protein may include (A)ₙ motifs. As used herein, the term "(A)ₙ motif" refers to an amino acid sequence mainly composed of alanine residues. The number of amino acid residues in the (A)ₙ motif may be an integer of 2 to 27, 2 to 20, 2 to 16, or 2 to 12. The proportion of the number of alanine residues with respect to the total number of amino acid residues in the (A)ₙ motif may be 40% or more, 60% or more, 70% or more, 80% or more, 83% or more, 85% or more, 86% or more, 90% or more, 95% or more, or 100%.

The artificial structural protein may be an artificial fibroin. As used herein, the term "artificial fibroin" refers to an artificially produced fibroin (synthetic fibroin). The artificial fibroin may be a fibroin having an amino acid sequence different from that of natural fibroin or a fibroin having the same amino acid sequence as that of natural fibroin. The artificial fibroin can be produced by known methods, and for example, WO 2021/187502 describes an example of the known methods.

The artificial fibroin may be a fibrous protein having a structure equivalent to that of natural fibroin. The artificial fibroin may include sequences similar to the repetitive sequences of natural fibroin. The "sequences similar to the repetitive sequences of fibroin" may be sequences included in natural fibroin or sequences similar to such sequences.

The artificial fibroin may be a fibroin having an amino acid sequence modified based on the amino acid sequence of natural fibroin (e.g., an artificial fibroin having an amino acid sequence that has been modified by altering a cloned gene sequence of natural fibroin) or a fibroin that is artificially designed without relying on natural fibroin (e.g., an artificial fibroin having a desired amino acid sequence, obtained by chemically synthesizing a nucleic acid encoding a designed amino acid sequence and then synthesizing the artificial fibroin using the thus-obtained nucleic acid). Fibroins having an amino acid sequence obtained by modifying the amino acid sequences of artificial fibroins are also encompassed in artificial fibroins, if their amino acid sequences are different from the amino acid sequence of natural fibroin. Examples of the artificial fibroin include artificial silk fibroins (fibroins having an amino acid sequence obtained by modifying the amino acid sequence of a silk protein produced by silkworms) and artificial spider silk fibroins (fibroins having an amino acid sequence obtained by modifying the amino acid sequence of a spider silk protein produced by spiders).

Natural fibroin based on which artificial fibroins are obtained may be fibroin produced by insects or spiders. Natural fibroin is a fibrous protein having a molecular weight of about 370,000 and composed of two subunits. In natural fibroin, the contents of glycine residues, alanine residues, serine residues, and tyrosine residues are high, and these amino acid residues account for nearly 90% of the total number of amino acid residues. Natural fibroin has a crystalline region in which amino acid residues having a relatively small side chain, e.g., glycine, alanine, and serine, are abundant and an amorphous region in which amino acid residues having a relatively large side chain, e.g., tyrosine, are present.

More specific examples of natural fibroin include fibroins whose sequence information is registered in the NCBI GenBank. For example, from information registered in the NCBI GenBank, it is possible to retrieve their sequence information by extracting, out of sequences containing INV as DIVISION, sequences with a keyword of spidroin, ampullate, fibroin, "silk and polypeptide", or "silk and protein" in DEFINITION, by extracting sequences having a specific product character string from CDS, and by extracting sequences having a specific character string in TISSUE TYPE from SOURCE.

The artificial fibroin may include a domain sequence represented by Formula 1: [(A)ₙ motif-REP]ₘ or Formula 2: [(A)ₙ motif-REP]ₘ-(A)ₙ motif. The artificial fibroin may have an amino acid sequence added to either one or each of the N-terminal side and the C-terminal side of the domain sequence (N-terminal sequence and C-terminal sequence). The N-terminal sequence and the C-terminal sequence are typically regions that do not include repetitive amino acid motifs characteristic of fibroin, but are not limited thereto. The N-terminal sequence and the C-terminal sequence are each composed of about 100 amino acid residues.

As used herein, the term "domain sequence" refers to an amino acid sequence that generates a crystalline region (typically corresponding to the (A)ₙ motif in the amino acid sequence) and an amorphous region (typically corresponding to REP in the amino acid sequence) characteristic of fibroin, which is an amino acid sequence represented by Formula 1: [(A)ₙ motif-REP]ₘ or Formula 2: [(A)ₙ motif-REP]ₘ-(A)ₙ motif. The (A)ₙ motif represents an amino acid sequence mainly includes alanine residues, and the number of amino acid residues therein may be 2 to 27. The number of amino acid residues in the (A)ₙ motif may be an integer of 2 to 20, 4 to 27, 4 to 20, 8 to 20, 10 to 20, 4 to 16, 8 to 16, or 10 to 16. The proportion of the number of alanine residues with respect to the total number of amino acid residues in the (A)ₙ motif may be 40% or more, 60% or more, 70% or more, 80% or more, 83% or more, 85% or more, 86% or more, 90% or more, 95% or more, or 100%. Of the plurality of (A)ₙ motifs present in the domain sequence, at least seven (A)ₙ motifs may be composed of only alanine residues. REP represents an amino acid sequence composed of 2 to 200 amino acid residues. Alternatively, REP may be an amino acid sequence composed of 10 to 200 amino acid residues. m represents an integer of 2 to 300, and may be an integer of 10 to 300. The plurality of (A)ₙ motifs may have amino acid sequences that are identical to or different from each other. The plurality of REPs may have amino acid sequences that are identical to or different from each other.

Examples of the artificial fibroin include the following artificial fibroins disclosed in WO 2021/187502: artificial fibroin derived from a large spinneret dragline silk protein produced in the major ampullate gland of spiders (first artificial fibroin); artificial fibroin having a domain sequence in which the content of glycine residues is reduced (second artificial fibroin); artificial fibroin having a domain sequence in which the content of the (A)ₙ motif is reduced (third artificial fibroin); artificial fibroin in which the content of glycine residues and the content of the (A)ₙ motif are reduced (fourth artificial fibroin); artificial fibroin having a domain sequence including a region locally having a high hydrophobicity index (fifth artificial fibroin); and artificial fibroin having a domain sequence in which the content of glutamine residues is reduced (sixth artificial fibroin). The definitions of the first to sixth artificial fibroins as disclosed in WO 2021/187502 are incorporated herein by reference.

The artificial fibroin may include a tag sequence at one or both of the N-terminus and the C-terminus thereof. This enables isolation, immobilization, detection, visualization, and the like of the artificial fibroin. PRT966 is the sixth artificial fibroin with a tag sequence.

The tag sequence may be, for example, an affinity tag that utilizes specific affinity (binding properties or affinity) to other molecules. A histidine tag (His-tag) is a specific example of the affinity tag. The His-tag is a short peptide in which about 4 to 10 histidine residues are lined up. Since the histidine tag has a property of specifically binding to metal ions, e.g., nickel, it can be used for isolating the artificial fibroin through chelating metal chromatography. The tag sequence may be, for example, an amino acid sequence set forth in SEQ ID NO: 8 (an amino acid sequence including a His-tag sequence and a hinge sequence).

Also, it is possible to use a tag sequence, e.g., glutathione-S-transferase (GST) that specifically binds to glutathione or a maltose binding protein (MBP) that specifically binds to maltose.

Further, it is also possible to use an "epitope tag" that utilizes an antigen-antibody reaction. By adding a peptide that exhibits antigenicity (epitope) as a tag sequence, an antibody against the epitope can bind to the artificial fibroin. Examples of the epitope tag include an HA (a peptide sequence of influenza virus hemagglutinin) tag, a myc tag, and a FLAG tag. By utilizing an epitope tag, purification of the artificial fibroin can be performed easily with high specificity.

Still further, it is also possible to use a tag sequence that can be cleaved by a specific protease. By subjecting a protein adsorbed via such a tag sequence to the protease treatment, it is possible to recover the artificial fibroin from which the tag sequence has been separated.

Table 1 shows some specific examples of the artificial fibroin.

**[Table 1]**

| | Content of alanine residues (%) | Content of glycine residues (%) | Content of serine residues (%) | Content of threonine residues (%) | Content of tyrosine residues (%) | Content of glutamine residues (%) | Content of lysine residues (%) |
|---|---|---|---|---|---|---|---|
| PRT799 (SEQ ID NO: 1) | 19.8 | 31.0 | 9.3 | 0 | 7.0 | 17.3 | 0.1 |
| PRT966 (SEQ ID NO: 2) | 19.7 | 31.2 | 9.4 | 0 | 7.1 | 0 | 0 |
| PRT918 (SEQ ID NO: 3) | 19.5 | 30.9 | 9.7 | 0 | 7.0 | 0 | 0 |
| PRT313 (SEQ ID NO: 4) | 25.7 | 36.0 | 8.9 | 0 | 6.4 | 8.2 | 0 |
| PRT1000 (SEQ ID NO: 5) | 19.4 | 30.8 | 9.6 | 0 | 7.0 | 0 | 0 |
| PRT1001 (SEQ ID NO: 6) | 19.3 | 30.7 | 9.6 | 0 | 6.9 | 0 | 0 |
| PRT587 (SEQ ID NO: 7) | 19.7 | 31.1 | 9.4 | 0 | 7.1 | 17.3 | 0 |

The artificial fibroin may be an artificial fibroin having at least two of the characteristics of the first artificial fibroin, second artificial fibroin, third artificial fibroin, fourth artificial fibroin, fifth artificial fibroin, and sixth artificial fibroin.

The molecular weight of the artificial fibroin is not limited to a particular value, and may be, for example, from 2 to 700 kDa. The molecular weight of the artificial fibroin may be, for example, 2 kDa or more, 3 kDa or more, 4 kDa or more, 5 kDa or more, 6 kDa or more, 7 kDa or more, 8 kDa or more, 9 kDa or more, 10 kDa or more, 20 kDa or more, 30 kDa or more, 40 kDa or more, 50 kDa or more, 60 kDa or more, 70 kDa or more, 80 kDa or more, 90 kDa or more, or 100 kDa or more, and may be 700 kDa or less, 600 kDa or less, 500 kDa or less, 400 kDa or less, less than 360 kDa, 300 kDa or less, or 200 kDa or less.

### Method for Recovering Carboxylic Acid

The method for recovering a carboxylic acid from a carboxylic acid aqueous solution is a method for recovering a carboxylic acid from a carboxylic acid aqueous solution Y containing a carboxylic acid, wherein the carboxylic acid is one that has been eluted from a protein solution containing a protein and the carboxylic acid and the concentration of the carboxylic acid is from 0.05 to 0.3 in terms of mass fraction.

The carboxylic acid aqueous solution Y is not limited to a particular carboxylic acid aqueous solution as long as it is an aqueous solution in which a carboxylic acid that has been eluted from a protein solution containing a protein and a solvent containing the carboxylic acid is contained at a concentration of 0.05 to 0.3 in terms of mass fraction, and for example, the above-described coagulation liquid Y can be suitably used.

The method for recovering the carboxylic acid from the carboxylic acid aqueous solution Y can be performed in the same manner as the above-described method for recovering the carboxylic acid from the coagulation liquid 11 (coagulation liquid Y), except that, in the above description, the term "coagulation liquid 11 (coagulation liquid Y)" is replaced with the term "carboxylic acid aqueous solution Y" and the term "coagulation liquid 11a (coagulation liquid Z)" is replaced with the term "carboxylic acid aqueous solution Z".

### Examples

The present invention is not limited to the following examples.

### 1. Concentration of Each Component

### (1) Water

The concentration of water was measured by gas chromatography under the following conditions.
Instrument: GC-2014 (Shimadzu Corporation)
Detector: thermal conductivity detector
Column: Chromosorb 101 (inner diameter: 2 mm, length: 1.83 m)
Temperature rise conditions for column: 90°C → raising the temperature at 10°C/min → 220°C (maintaining the temperature for 47 minutes)
Inlet condition: 250°C
Carrier gas flow rate: 4 mL/min

### (2) Carboxylic acid

When a concentration of a carboxylic acid was 0.5 or less in terms of mass fraction, the concentration of a carboxylic acid was measured by neutralization titration performed using an automatic potentiometric titrator under the following conditions.
Titrator: AT-710 (Kyoto Electronics Manufacturing Co., Ltd.)
Titration reagent: 0.1 mol/L ethanolic potassium hydroxide aqueous solution (FUJIFILM Wako Pure Chemical Corporation)

When the concentration of a carboxylic acid was more than 0.5 in terms of mass fraction, the concentration of the remainder determined by subtracting the concentrations of other components was regarded as the concentration of a carboxylic acid.

### (3) Other volatile components

The concentrations of other volatile components were measured by gas chromatography under the following conditions.
Instrument: GC-2014 (Shimadzu Corporation)
Detector: hydrogen flame ion detector
Column: G-100 (inner diameter: 1.2 mm, length: 40 m, film thickness: 3.0 µm)
Temperature rise conditions for column: 80°C → raising the temperature at 10°C/min → 240°C (maintaining the temperature for 44 minutes)
Inlet condition: 250°C
Carrier gas flow rate: 15 ml/min

### (4) Nonvolatile components

About 5.0 g of a sample solution was added to a φ 100 ml petri dish, and the sample solution in the petri dish was allowed to stand still under a reduced pressure of about 1.3 kPa in a vacuum dryer set at 140°C for 24 hours. The concentration of nonvolatile components was determined based on the mass of the sample solution after being allowed to stand still and the mass of the sample solution.

### 2. Production of Formed article (Fiber)

### Example 1

### Spinning

Dry powder of artificial spider silk fibroin was dissolved in formic acid to obtain a protein solution to be used as a spinning dope. According to wet spinning in which the spinning dope was spun into a coagulation liquid X (sodium sulfate aqueous solution) and the formic acid was eluted from the spinning dope into the coagulation liquid, a formed article in the form of a fiber containing the coagulated artificial spider silk fibroin was formed. The thus-formed fiber was dried and wound. The concentration (mass fraction) of each component in a coagulation liquid Y after the wet spinning was as follows.

### Coagulation liquid Y (before treatment)

- Formic acid: 0.2
- Sodium sulfate: 0.14
- Impurities: 0.0005
- Water: the remainder

### Recovery of Formic Acid

Purified formic acid was recovered from the coagulation liquid Y after the wet spinning through the following First Step, Second Step, and Third Step using the production apparatus shown in FIG. 2.

### (First Step)

A packing-type extraction tower having a tower diameter of 65 mm and a height equivalent to three theoretical plates was used as a liquid-liquid extraction section 51. A mixed liquid containing tributyl phosphate (TBP, boiling point: 293.0°C) as an extraction solvent and decane (boiling point: 174.1°C) as a diluent at a mass ratio (TBP:decane) of 3:1 was prepared as a prepared extractant 40 (prepared extractant I). The specific gravity of the prepared extractant 40 was 0.89 at 20°C. A coagulation liquid 11 (coagulation liquid Y) and the prepared extractant 40, which were at 40°C, were fed to the liquid-liquid extraction section 51, in which a dispersed phase containing the coagulation liquid 11 and a continuous phase containing the prepared extractant 40 were brought into liquid-liquid contact with each other. The feed flow rate of the coagulation liquid 11 was 40 kg/h, and the feed flow rate of the prepared extractant 40 was 130 kg/h. 320 kg (formic acid: 64 kg) of the coagulation liquid Y was brought into liquid-liquid contact with the prepared extractant I through 8 hours of treatment.

A prepared extractant 40a (light liquid, prepared extractant II) containing the formic acid and the water was extracted from a nozzle provided on the upper side of the liquid-liquid extraction section 51. A coagulation liquid 11a (heavy liquid, coagulation liquid Z) remaining after the formic acid had been removed was extracted from a nozzle provided on the bottom side of the liquid-liquid extraction section 51. The coagulation liquid 11a (coagulation liquid Z) mainly contained the water. Since precipitates were observed at the interface between an upper layer and a lower layer during extraction, a middle layer was removed separately. More specifically, a middle layer 41 containing impurities precipitated at the interface in the liquid-liquid contact was extracted from a nozzle provided in a lower part of the liquid-liquid extraction section 51. The middle layer 41 was centrifuged to remove the impurities.

As a result of treating 96% of the total volume excluding a portion partially held in the extraction tower and a portion removed as the middle layer, the concentration (mass fraction) of each component in 1081.90 kg of the prepared extractant 40a (prepared extractant II) extracted from the liquid-liquid extraction section 51 was as follows. The amount of the formic acid contained in the prepared extractant 40a after the liquid-liquid contact with the coagulation liquid was 61.15 kg, and the formic acid recovery rate was found to be 99.3 mass%.

### Prepared extractant after the First Step (prepared extractant II)

Formic acid: 0.057
Decane: 0.231
Water: 0.018
Sodium sulfate: trace amount (TR)
TBP: the remainder

The concentration (mass fraction) of each component in 226.95 kg of the heavy liquid (coagulation liquid 11a) extracted from the liquid-liquid extraction section was as follows.

### Coagulation liquid after the First Step (coagulation liquid Z)

Sodium sulfate: 0.19
Formic acid: 0.002
TBP: 0.0008
Decane: less than 0.00003
Water: the remainder

In the First Step, the distribution ratio D of the formic acid was 28.5 (= the concentration of the formic acid in the prepared extractant II/the concentration of the formic acid in the coagulation liquid Z = 0.057/0.002).

### (Second Step)

A distillation tower 52 for the Second Step was a continuous-type plate tower with the number of actual plates being 8. To a feed plate positioned at the fourth stage from the top of the distillation tower 52, 1080 kg of the prepared extractant 40a (prepared extractant II) after the First Step was fed at a feed rate of 200 kg/h. Through a distillation operation of about 5 hours, the decane and the water were azeotropically distilled off. The distillation operation was performed under the following conditions: a reduced pressure of 13.3 kPa; a tower bottom temperature of 118°C to 120°C; and a tower top temperature of 52°C to 53°C. 50 kg of the prepared extractant 40a was consumed for holding loss in distillation and sampling, and 95% of the total fed amount was treated. The decane and water that have been distilled off were introduced from the top of the distillation tower 52 into a decanter 61, in which a mixed liquid generated through condensation was separated into a diluent layer 42 mainly containing the decane and an aqueous layer 43 mainly containing the water. The aqueous layer 43 passed through a return line and was added to a coagulation liquid 11 (coagulation liquid Y), which was to be introduced into the liquid-liquid extraction section 51 for the First Step. The diluent layer 42 was returned to the distillation tower 52 as a reflux with a reflux ratio of 2 via a reflux line. From the bottom of the distillation tower 52, a prepared extractant 40b (prepared extractant III) remaining after the removal of most of the water was extracted at a rate of 180 kg/h. The concentration (mass fraction) of each component in the extracted prepared extractant 40b was as follows. No precipitates were observed at the bottom of the distillation tower during the distillation operation.

### Prepared extractant III (after the Second Step)

Formic acid: 0.056
Decane: 0.236
Water: less than 0.0003
TBP: the remainder

### (Third Step)

A continuous-type packed tower with the number of theoretical plates being 8 was used as a distillation tower 53 for the Third Step. To a feed plate positioned at the fourth stage from the top of the distillation tower 53, 1000 kg of the prepared extractant 40b (prepared extractant III) remaining after the removal of most of the water in the second step was fed at a feed rate of 180 kg/h. Through a distillation operation of about 6 hours, the decane and the formic acid in the prepared extractant 40b were azeotropically distilled off. The distillation operation was performed under the following conditions: a reduced pressure of 11.3 kPa; a tower bottom temperature of 130°C to 131°C; and a tower top temperature of 85°C to 90°C. 40 kg of the prepared extractant 40b was consumed for holding loss in distillation and sampling, and 96% of the total fed amount was treated. The decane and formic acid that had been distilled off were introduced from the top of the distillation tower 53 into a decanter 62, in which a mixed liquid generated through condensation was separated into a diluent layer 44 mainly containing the decane and a layer of purified carboxylic acid 100 mainly containing the formic acid. The diluent layer 44 was returned to the distillation tower 53 as a reflux with a reflux ratio of 4 via a reflux line. From the bottom of the distillation tower 53, a prepared extractant 40c (prepared extractant IV) remaining after the removal of the formic acid and the water was discharged at a rate of 150 kg/h. The discharged prepared extractant 40c passed through a return line and was added to the prepared extractant 40 (prepared extractant I) before being introduced into the liquid-liquid extraction section 51.

The concentration (mass fraction) of each component in 60 kg of the recovered purified carboxylic acid containing the formic acid was as follows.

### Purified carboxylic acid (after the Third Step)

Formic acid: 0.99
Water: 0.003
TBP: 0.003
Decane: 0.002

The concentration (mass fraction) of each component in the prepared extractant 40c remaining after the removal of the formic acid and the water and discharged from the bottom of the tower in the Third Step was as follows.

### Prepared extractant IV (after the Third Step)

Formic acid: 0.0001
Decane: 0.250
Water: 0.0000
TBP: the remainder

### (Fourth Step)

A continuous-type packed tower with the number of theoretical plates being 7 was used as a distillation tower 54 for the Fourth Step. 51 kg of the purified carboxylic acid 100 obtained in the Third Step was fed into the distillation tower 54 at a feed rate of 45 kg/h. The distillation operation was performed under total reflux conditions with a reduced pressure of 30 kPa, a tower bottom temperature of 68°C to 70°C, and a tower top temperature of 63°C to 64°C. 5 kg of the purified carboxylic acid 100 was consumed for holding loss in distillation and sampling, and 90% of the total fed amount was treated. A heavy liquid 45 containing the water and TBP was extracted from the bottom of the distillation tower 54, and a light liquid 46 containing the decane was extracted from the top of the distillation tower 54. The purified carboxylic acid 100a was recovered from a plate at a middle stage of the distillation tower 54. The concentration (mass fraction) of each component in the recovered purified carboxylic acid 100a was as follows.

### Purified carboxylic acid (after the Fourth Step)

Formic acid: 0.999
Water: 0.001

### (Fifth Step)

A continuous-type plate tower with the number of actual plates being 6 was used as a distillation tower 55 for the Fifth Step. 226.95 kg of the heavy liquid (coagulation liquid 11a) extracted from the liquid-liquid extraction section 51 in the First Step was fed into a feed plate positioned at the third stage from the top of the distillation tower 55 at a feed rate of 60 kg/h. The distillation operation was performed under total reflux conditions with a reduced pressure of 101.3 kPa, a tower bottom temperature of 102°C to 103°C, and a tower top temperature of 100°C. 25 kg of the heavy liquid was consumed for holding loss in distillation and sampling, and 89% of the total fed amount was treated. A mixed liquid 48 was extracted from the bottom of the distillation tower 55 at a rate of 55 kg/h. The concentration (mass fraction) of each component in the mixed liquid 48 was as follows. It was confirmed that the mixed liquid 48 was reusable as a sodium sulfate aqueous solution for industrial use.
Sodium sulfate: 0.191
Formic acid: 0.002
Water: the remainder

A distillate 47 was extracted from the top of the distillation tower 55. The concentration (mass fraction) of each component in the distillate 47 was as follows. It was confirmed that the distillate 47 may be added to the coagulation liquid 11, which is to be introduced into the liquid-liquid extraction section 51 in the First Step.
TBP: 0.0015
Decane: 0.06
Water: the remainder

### Example 2

As samples simulating the coagulation liquid Y after the wet spinning, coagulation liquids containing each component at the following concentration (mass fraction) were prepared.
Formic acid: 0.2
Sodium sulfate: 0, 0.01, 0.1, or 0.2
Water: the remainder

Each of the thus-prepared coagulation liquids was mixed with a prepared extractant containing TBP and decane at a mass ratio of (TBP : decane =) 2:1 in a separatory funnel. The mass ratio between the coagulation liquid and the prepared extractant was 1:1. The separatory funnel was sufficiently shaken at 23°C. The separatory funnel was allowed to stand still for 5 minutes, and thereafter, a coagulation liquid layer (heavy liquid) and a prepared extractant layer (light liquid) formed in the separatory funnel were extracted. The concentration (mass fraction) of the water in the extracted prepared extractant layer (light liquid) is shown in Table 2. Further, the layer separability was also evaluated according to the following evaluation criteria, based on the time elapsed from the time point at which the separatory funnel started to stand still to until two uniform layers were formed with no suspended solids at the interface.

### Evaluation Criteria for Layer Separability

A: 30 seconds or less
B: more than 30 seconds and 2 minutes or less
C: more than 2 minutes

**[Table 2]**

| Coagulation liquid | Prepared extractant layer | Layer separability |
|---|---|---|
| Concentration of sodium sulfate (mass fraction) | Concentration of water (mass fraction) | |
| 0 | 0.029 | B |
| 0.01 | 0.029 | B |
| 0.1 | 0.027 | A |
| 0.2 | 0.024 | A |

The results shown in Table 2 confirm that the presence of an inorganic salt in a coagulation liquid tends to suppress the migration of water to a prepared extractant layer and to improve the layer separability.

### Examples 3 to 8

As a sample simulating the coagulation liquid Y after the wet spinning, a coagulation liquid containing each component at the following concentration (mass fraction) was prepared.
Formic acid: 0.18
Sodium sulfate: 0.15
Impurities: 0.0005
Water: the remainder

Prepared extractants with the compositions shown in Table 3 were prepared. Each of the prepared extractants was mixed with the coagulation liquid in a separatory funnel. The mass ratio between the coagulation liquid and the prepared extractant was 1:1. The separatory funnel was sufficiently shaken at 23°C. The separatory funnel was allowed to stand still for 5 minutes, and thereafter, a coagulation liquid layer (heavy liquid) and a prepared extractant layer (light liquid) formed in the separatory funnel were extracted. The concentrations (mass fractions) of the formic acid and the water in the extracted prepared extractant layer (light liquid) are shown in Table 3. The layer separability was evaluated according to the same evaluation criteria as in Example 2. The distribution ratio shown in Table 3 is a value calculated as per the following equation. Distribution ratio = (a concentration of the formic acid in the prepared extractant layer)/(a concentration of the formic acid in the coagulation liquid layer)

**[Table 3]**

| | Prepared extractant | | | Prepared extractant layer | | Distribution ratio | Layer separability |
|---|---|---|---|---|---|---|---|
| | Extraction solvent | Diluent | Extraction solvent : diluent (mass ratio) | Concentration of formic acid | Concentration of water | | |
| Comparative Example 1 | Trioctylamine | None | - | 0.14 | 0.06 | 21.65 | C |
| Example 3 | Trioctylamine | Toluene | 1:1 | 0.13 | 0.02 | 2.52 | C |
| Example 4 | Trioctylamine | Octane | 2:1 | 0.14 | 0.02 | 3.81 | B |
| Comparative Example 2 | Dibutylformamide | None | - | 0.12 | 0.08 | 1074 | B |
| Example 5 | Dibutylformamide | Octane | 1:1 | 0.08 | 0.02 | 0.59 | A |
| Example 6 | Dibutylformamide | Decane | 1:1 | 0.08 | 0.02 | 0.58 | A |
| Comparative Example 3 | Tributyl phosphate | None | - | 0.10 | 0.09 | 0.91 | B |
| Example 7 | Tributyl phosphate | Octane | 3:1 | 0.08 | 0.03 | 0.61 | B |
| Example 8 | Tributyl phosphate | Decane | 3:1 | 0.08 | 0.03 | 0.62 | A |

### Description of Reference Numerals

- 1: Extruder
- 2: Coagulation device
- 3: Washing device
- 4: Drying device
- 5: Wound roll
- 6: Protein solution
- 7: Storage tank
- 8: Gear pump
- 9: Nozzle
- 10: Spinning apparatus
- 11, 11a: Coagulation liquid
- 12: Washing liquid
- 13: First nip roller
- 14: Second nip roller
- 18a to 18g: Yarn guide
- 17: Drying oven
- 19: Air gap
- 20: Coagulation bath
- 21: Washing bath
- 22: Yarn path (in drying oven)
- 36: Fiber (formed article)
- 40, 40a, 40b, 40c: Prepared extractant
- 41: Middle layer
- 42, 44: Diluent layer
- 43: Aqueous layer
- 45: Heavy liquid
- 46: Light liquid
- 47: Mixed liquid
- 48: Distillate
- 51: Liquid-liquid extraction section
- 52, 53, 54, 55: Distillation tower
- 61,62: Decanter
- 100, 100a: Purified carboxylic acid
- S1: First step
- S2: Second step
- S3: Third step
- S4: Fourth step
- S5: Fifth step

## Claims

1. A method for producing a formed article containing a protein, comprising:
introducing a protein solution comprising a protein and a solvent comprising a carboxylic acid into a coagulation liquid X comprising water,
eluting the carboxylic acid in the protein solution into the coagulation liquid X, thereby forming a formed article comprising the protein; and
recovering the carboxylic acid from a coagulation liquid Y in which the formed article comprising the protein has been formed,
wherein recovering the carboxylic acid from the coagulation liquid Y comprises:
a first step of bringing the coagulation liquid Y into liquid-liquid contact with a prepared extractant I comprising: an extraction solvent that dissolves the carboxylic acid and a diluent that is completely compatible with the extraction solvent, thereby forming a coagulation liquid Z and a prepared extractant II,
wherein in the coagulation liquid Z, a concentration of the extraction solvent and a concentration of the diluent are each less than 0.001 mass fraction and a concentration of the carboxylic acid is less than 0.005 mass fraction, and
in the prepared extractant II, 90 mass% or more of the carboxylic acid present in the coagulation liquid Y is dissolved and a concentration of water is less than 0.05 mass fraction;
a second step of forming a diluent layer comprising the diluent and an aqueous layer comprising water while azeotropically distilling off the diluent and the water from the prepared extractant II and also recovering a prepared extractant III that is a remainder comprising the carboxylic acid; and
a third step of obtaining a purified carboxylic acid comprising the carboxylic acid that has been purified through a distillation operation including distilling off the carboxylic acid from the prepared extractant III recovered in the second step,
wherein the extraction solvent is a poorly water-soluble organic solvent having a boiling point higher than a boiling point of the diluent at atmospheric pressure,
the diluent is:
a first hydrophobic solvent that forms an azeotropic mixture with water, has a water concentration of 0.2 or more mass fraction and has a boiling point higher than a boiling point of water and a boiling point of the carboxylic acid at atmospheric pressure, wherein the first hydrophobic solvent does not form an azeotropic mixture with the carboxylic acid; or
a second hydrophobic solvent that forms an azeotropic mixture with water, has a water concentration of 0.2 or more mass fraction and has a boiling point higher than a boiling point of water and a boiling point of the carboxylic acid at atmospheric pressure,
wherein the second hydrophobic solvent forms an azeotropic mixture with the carboxylic acid, and
a mixed liquid comprising the second hydrophobic solvent and the carboxylic acid at any ratio separates into a layer comprising the second hydrophobic solvent and a layer comprising the carboxylic acid,
when the diluent is the second hydrophobic solvent, in the third step, the distillation operation comprises azeotropically distilling off the carboxylic acid and the diluent from the prepared extractant III, and the purified carboxylic acid and the diluent layer comprising the diluent are formed by the distillation operation, and
in the purified carboxylic acid, a concentration of water, a concentration of the extraction solvent, and a concentration of the diluent are each less than 0.01 mass fraction.

2. The method according to claim 1, wherein
in the first step, a distribution ratio D of the carboxylic acid represented by the following equation is 0.3 or more: D = (a concentration of the carboxylic acid in the prepared extractant II)/(the concentration of the carboxylic acid in the coagulation liquid Z).

3. The method according to claim 1 or 2, wherein
a concentration of the carboxylic acid in the coagulation liquid Y is from 0.05 to 0.3 mass fraction, and
a concentration of the carboxylic acid in the purified carboxylic acid is 0.99 or more mass fraction.

4. The method according to claim 1 or 2, wherein
the diluent layer formed in the second step is added to the prepared extractant II.

5. The method according to claim 1 or 2, wherein
the aqueous layer formed in the second step is added to the coagulation liquid Y

6. The method according to claim 1 or 2, wherein
a prepared extractant IV remaining after the carboxylic acid has been distilled off from the prepared extractant III in the third step is added to the prepared extractant I.

7. The method according to claim 1 or 2, wherein
the diluent is the second hydrophobic solvent, and the diluent layer formed in the third step is added to the prepared extractant III.

8. A method for recovering a carboxylic acid, comprising:
preparing a carboxylic acid aqueous solution Y comprising a carboxylic acid, the carboxylic acid having been eluted from a protein solution comprising a protein and a solvent comprising the carboxylic acid and a concentration of the carboxylic acid being from 0.05 to 0.3 mass fraction; and
recovering the carboxylic acid from the carboxylic acid aqueous solution Y,
wherein recovering the carboxylic acid from the carboxylic acid aqueous solution Y comprises:
a first step of bringing the carboxylic acid aqueous solution Y into liquid-liquid contact with a prepared extractant I comprising: an extraction solvent that dissolves the carboxylic acid and a diluent that is completely compatible with the extraction solvent, thereby forming a carboxylic acid aqueous solution Z and a prepared extractant II,
wherein in the carboxylic acid aqueous solution Z, a concentration of the extraction solvent and a concentration the diluent are each less than 0.001 mass fraction and a concentration of the carboxylic acid is less than 0.005 mass fraction, and
in the prepared extractant II, 90 mass% or more of the carboxylic acid present in the carboxylic acid aqueous solution Y is dissolved and a concentration of water is less than 0.05 mass fraction;
a second step of forming a diluent layer comprising the diluent and an aqueous layer comprising water while azeotropically distilling off the diluent and the water from the prepared extractant II and also recovering a prepared extractant III that is a remainder comprising the carboxylic acid; and
a third step of obtaining a purified carboxylic acid comprising the carboxylic acid that has been purified through a distillation operation including distilling off the carboxylic acid from the prepared extractant III recovered in the second step,
wherein the extraction solvent is a poorly water-soluble organic solvent having a boiling point higher than a boiling point of the diluent at atmospheric pressure,
the diluent is:
a first hydrophobic solvent that forms an azeotropic mixture with water, has a water concentration of 0.2 or more mass fraction and has a boiling point higher than a boiling point of water and a boiling point the carboxylic acid at atmospheric pressure, wherein the first hydrophobic solvent does not form an azeotropic mixture with the carboxylic acid; or
a second hydrophobic solvent that forms an azeotropic mixture with water, has a water concentration of 0.2 or more mass fraction and has a boiling point higher than a boiling point of water and a boiling point the carboxylic acid at atmospheric pressure, wherein the second hydrophobic solvent forms an azeotropic mixture with the carboxylic acid, and a mixed liquid comprising the second hydrophobic solvent and the carboxylic acid at any ratio separates into a layer comprising the second hydrophobic solvent and a layer comprising the carboxylic acid,
when the diluent is the second hydrophobic solvent, in the third step, the distillation operation comprises azeotropically distilling off the carboxylic acid and the diluent from the prepared extractant III, and the purified carboxylic acid and the diluent layer comprising the diluent are formed by the distillation operation, and
in the purified carboxylic acid, a concentration of water, a concentration of the extraction solvent, and a concentration of the diluent are each less than 0.01 mass fraction and a concentration of the carboxylic acid is 0.99 or more mass fraction.

9. The method according to claim 8, wherein
in the first step, a distribution ratio D of the carboxylic acid represented by the following equation is 0.3 or more: D = (a concentration of the carboxylic acid in the prepared extractant II)/(the concentration of the carboxylic acid in the carboxylic acid aqueous solution Z).

10. The method according to claim 8 or 9, wherein
the diluent layer formed in the second step is added to the prepared extractant II.

11. The method according to claim 8 or 9, wherein
the aqueous layer formed in the second step is added to the carboxylic acid aqueous solution Y

12. The method according to claim 8 or 9, wherein
a prepared extractant IV remaining after the carboxylic acid has been distilled off from the prepared extractant III in the third step is added to the prepared extractant I.

13. The method according to claim 8 or 9, wherein
the diluent is the second hydrophobic solvent, and the diluent layer formed in the third step is added to the prepared extractant III.
